# EUROPEAN PATENT APPLICATION

(11) **EP 4 393 542 A1**
(43) Date of publication of application: **03.07.2024**
(21) Application number: 22861276.8
(22) Date of filing: 19.08.2022
(51) Int. Cl.: A61N 5/067, A61B 18/24, G02B 6/00

(54) **IRRADIATION PROBE AND IRRADIATION PROBE SYSTEM**

(30) Priority: 27.08.2021 JP 2021139393
(71) Applicant: FURUKAWA ELECTRIC CO., LTD., Tokyo 100-8322 (JP)
(72) Inventor: IWAMA, Masaki, Tokyo 100-8322 (JP)
(74) Representative: SSM Sandmair
(86) International application number: PCT/JP2022/031414
(87) International publication number: WO 2023/026988

(57) **Abstract**

An irradiation probe is, for example, an irradiation probe in which a plurality of optical fibers are bundled together, each of the optical fibers having, as at least a partial section in a longitudinal direction, a leakage section that outputs leakage light radially outward. Each of the optical fibers has directivity in which intensity of leakage light in a specific radial direction is higher than intensity of leakage light in another radial direction in a cross section intersecting an axial direction of the leakage section. The optical fibers are disposed apart from a central axis of the irradiation probe in radial directions different from each other, and the optical fibers are bundled together in a posture in which leakage light to the specific radial direction from the leakage section is directed radially outward of the irradiation probe.

## Description

### Field

The present invention relates to an irradiation probe and an irradiation probe system.

### Background

Conventionally, known examples of a medical probe that can be used for photodynamic therapy (PDT) or photodynamic diagnosis (PDD) include a probe that emits laser light from a side surface of the probe to the surrounding area and a probe that emits laser light from a side surface to a specific direction (for example, see Patent Literature 1).

### Citation List

### Patent Literature

Patent Literature 1: JP 3675482 B2

### Summary

### Technical Problem

In the case of using the probe that emits light entirely to the surrounding area from the side surface disclosed in Patent Literature 1, the light may be unnecessarily applied to, for example, even a region in the living body where the light is not required to be applied originally or is not to be applied.

Further, in the case of using the probe that emits light only in a specific direction from the side surface disclosed in Patent Literature 1, if the light is applied to, for example, a region in the living body different from a region originally intended to be irradiated, it is necessary to change the rotational posture around the axis of the probe. This change in rotational posture of the probe is not easy and is labor-intensive.

Therefore, one of the problems to be solved by the present invention is to achieve an improved novel irradiation probe and irradiation probe system that are, for example, capable of switching the irradiation direction of light from a side surface of the probe around the axis of the probe.

### Solution to Problem

An irradiation probe according to the present invention, for example, includes: a plurality of optical fibers bundled together, each of the optical fibers including, as at least a partial section in a longitudinal direction, a leakage section configured to output leakage light radially outward, wherein each of the optical fibers has directivity in which intensity of leakage light in a specific radial direction is higher than intensity of leakage light in another radial direction in a cross section intersecting an axial direction of the leakage section, the optical fibers are disposed apart from a central axis of the irradiation probe in radial directions different from each other, and the optical fibers are bundled together in a posture in which leakage light to the specific radial direction from the leakage section is directed radially outward of the irradiation probe.

An irradiation probe according to the present invention, for example, includes: a plurality of optical fibers bundled together, each of the optical fibers including, as at least a partial section in a longitudinal direction, a leakage section configured to output leakage light radially outward, wherein the irradiation probe includes a reflective member placed at least radially inward of the irradiation probe with respect to the optical fiber and configured to reflect the leakage light from the optical fiber, each of the optical fibers has directivity in which intensity of leakage light in a specific radial direction is higher than intensity of leakage light in another radial direction in a cross section intersecting an axial direction of the leakage section, the optical fibers are disposed apart from a central axis of the irradiation probe in radial directions different from each other, and the optical fibers are bundled together in a posture in which leakage light to the specific radial direction from the leakage section is directed radially inward of the irradiation probe.

In the irradiation probe, in the leakage section, at least one of the optical fibers may include a scattering region in which light is scattered in a predetermined range in a circumferential direction of the optical fiber.

An outer surface of the optical fiber in the scattering region may be a convex curved surface in which an average radius of curvature in the scattering region is equal to or greater than a radius of a general region different from the scattering region, a plane intersecting a radial direction of the optical fiber, or a concave curved surface that is concave radially inward.

The optical fibers may be disposed apart from a central axis of the irradiation probe in radial directions different from each other, and the irradiation probe may include a shielding member that prevents the leakage light from the leakage section of each of the optical fibers from traveling radially inward or a circumferential direction of the irradiation probe.

An irradiation probe according to the present invention, for example, includes: a plurality of optical fibers bundled together, each of the optical fibers including, as at least a partial section in a longitudinal direction, a leakage section configured to output leakage light radially outward, wherein the optical fibers are disposed apart from a central axis of the irradiation probe in radial directions different from each other, and the irradiation probe includes a shielding member configured to prevent the leakage light from the leakage section of each of the optical fibers from traveling radially inward or a circumferential direction of the irradiation probe.

In the irradiation probe, the shielding member may be configured to reflect the leakage light.

In the irradiation probe, the shielding member may include an intervening portion placed between two optical fibers of the plurality of optical fibers in a circumferential direction of the irradiation probe.

In the irradiation probe, the shielding member may include an opaque fiber that does not transmit leakage light.

In the irradiation probe, the shielding member may include, as the opaque fiber, a first opaque fiber placed radially inward of the irradiation probe with respect to the optical fiber or placed between two optical fibers of the plurality of optical fibers in a circumferential direction of the irradiation probe.

In the irradiation probe, the shielding member may include, as the opaque fiber: two first opaque fibers adjacent to each other; and a second opaque fiber placed on a side close to the optical fiber with respect to a boundary between the two first opaque fibers.

In the irradiation probe, the shielding member may include a metal member.

In the irradiation probe, the metal member may have conductivity.

In the irradiation probe, the shielding member may include: a cover as the metal member; and a core member surrounded by the cover and made of a material having an elastic modulus smaller than an elastic modulus of the metal member.

In the irradiation probe, the shielding member may include an extending portion extending in a radial direction of the irradiation probe at least in the leakage section.

In the irradiation probe, a plurality of extending portions may be integrated radially inward of the irradiation probe as the extending portion.

In the irradiation probe, the shielding member may include a sleeve configured to partially cover an outer periphery of the optical fiber at least in the leakage section and, extend in the longitudinal direction, the sleeve including an opening opened radially outward of the irradiation probe.

In the irradiation probe, the irradiation probe may include a projected portion projected by projection light of the irradiation probe, the projected portion being configured such that, when the projection light is projected in a radial direction of the irradiation probe, a projected shape by the projection light varies depending on a rotational posture around a central axis of the irradiation probe.

In the irradiation probe, the projected portion may include at least two portions that are separated from each other in the longitudinal direction and are separated from each other in a circumferential direction of the irradiation probe at a central angle different from 0° or 180° when viewed in the longitudinal direction.

In the irradiation probe, the optical fiber may include an outer scattering portion placed radially outward of the irradiation probe in the leakage section to scatter light.

In the irradiation probe, the optical fiber may include an inner scattering portion placed radially inward of the irradiation probe in the leakage section to scatter light.

The irradiation probe may include a core wire extending in a longitudinal direction of the irradiation probe, wherein the plurality of optical fibers may be arranged along an outer periphery of the core wire.

In the irradiation probe, a diameter of the core wire may be larger than a diameter of the optical fiber.

In the irradiation probe, the plurality of optical fibers may be at least partially accommodated in a recess provided in the core wire.

In the irradiation probe, the core wire may include a portion made of a synthetic resin material.

In the irradiation probe, the core wire may include a scattering body.

An irradiation probe system according to the present invention, for example, include: the irradiation probe; a light source; and a switching mechanism configured to selectively input light from the light source to at least one of the plurality of optical fibers.

An irradiation probe system according to the present invention, for example, include: an irradiation probe including a plurality of optical fibers bundled together, the plurality of optical fibers being arranged side by side in a circumferential direction of the irradiation probe and configured to leak light radially outward from an outer surface of each of the plurality of optical fibers in a leakage section in a longitudinal direction; a light source; and a switching mechanism configured to selectively input light from the light source to at least one of the plurality of optical fibers.

The irradiation probe system may include a plurality of light sources as the light source.

The irradiation probe system may include a control mechanism configured to control at least one of the light source and the switching mechanism so that light from the light source is intermittently input to the optical fiber.

In the irradiation probe system, the switching mechanism may be configured to operate so that the optical fiber to which light from the light source is input is sequentially switched over time in a circumferential direction of the irradiation probe.

### Advantageous Effects of Invention

According to the present invention, for example, an improved novel irradiation probe system and irradiation probe can be achieved.

### Brief Description of Drawings

FIG. 1 is an exemplary and schematic configuration diagram of an irradiation probe system according to a first embodiment.
FIG. 2 is an exemplary and schematic cross-sectional view of an irradiation probe according to the first embodiment.
FIG. 3 is an exemplary and schematic cross-sectional view of a part of an optical fiber of the irradiation probe according to the first embodiment.
FIG. 4 is an exemplary block diagram of an irradiation probe system according to an embodiment.
FIG. 5 is an exemplary and schematic cross-sectional view of an irradiation probe illustrating an example of a temporal change in a light irradiation state by the irradiation probe system of the embodiment.
FIG. 6 is an exemplary and schematic cross-sectional view of an irradiation probe illustrating an example of a temporal change in a light irradiation state by the irradiation probe system of the embodiment.
FIG. 7 is an exemplary schematic diagram of a switching mechanism of an irradiation probe system according to a second embodiment.
FIG. 8 is an exemplary schematic diagram of the switching mechanism of the irradiation probe system according to the second embodiment, and is a diagram illustrating a state different from that in FIG. 7.
FIG. 9 is an exemplary schematic diagram of the switching mechanism of the irradiation probe system according to the second embodiment, and is a diagram illustrating a state different from those in FIGS. 7 and 8.
FIG. 10 is a schematic cross-sectional view of a modification of a scattering region of an irradiation probe of the embodiment.
FIG. 11 is a schematic cross-sectional view of a modification of the scattering region of the irradiation probe of the embodiment.
FIG. 12 is a schematic cross-sectional view of a modification of the scattering region of the irradiation probe of the embodiment.
FIG. 13 is a schematic side view of a modification of the scattering region of the irradiation probe of the embodiment.
FIG. 14 is a schematic cross-sectional view of a modification of the scattering region of the irradiation probe of the embodiment.
FIG. 15 is an exemplary and schematic cross-sectional view of an irradiation probe of a modification to the embodiment.
FIG. 16 is an exemplary and schematic cross-sectional view of an irradiation probe of a modification to the embodiment.
FIG. 17 is a partially enlarged view of FIG. 16.
FIG. 18 is an exemplary and schematic cross-sectional view of a part of an irradiation probe of a modification to the embodiment.
FIG. 19 is an exemplary and schematic cross-sectional view of a part of an irradiation probe of a modification to the embodiment.
FIG. 20 is an exemplary and schematic cross-sectional view of a part of an irradiation probe of a modification to the embodiment.
FIG. 21 is an exemplary and schematic cross-sectional view of a part of an irradiation probe of a modification to the embodiment.
FIG. 22 is an exemplary and schematic cross-sectional view of a part of an irradiation probe of a modification to the embodiment.
FIG. 23 is an exemplary and schematic cross-sectional view of an irradiation probe of a modification to the embodiment.
FIG. 24 is an exemplary and schematic cross-sectional view of an irradiation probe of a modification to the embodiment.
FIG. 25 is an exemplary and schematic cross-sectional view of an irradiation probe of a modification to the embodiment.
FIG. 26 is an exemplary and schematic cross-sectional view of an irradiation probe of a modification to the embodiment.
FIG. 27 is an exemplary and schematic cross-sectional view of an irradiation probe of a modification to the embodiment.
FIG. 28 is an exemplary and schematic cross-sectional view of a part of an optical fiber of a modification to the embodiment.
FIG. 29 is an exemplary and schematic side view of a shielding member provided in an end portion of an irradiation probe of a modification to the embodiment.
FIG. 30 is an exemplary and schematic side view of the shielding member of FIG. 29 in which a rotational posture around a central axis is different from that of FIG. 29.
FIG. 31 is an exemplary and schematic diagram illustrating a shielding member provided in an end portion of an irradiation probe of a modification to the embodiment.
FIG. 32 is an exemplary and schematic diagram illustrating the shielding member of FIG. 31 in which a rotational posture around a central axis is different from that of FIG. 31.
FIG. 33 is an exemplary and schematic diagram illustrating a shielding member provided in an end portion of an irradiation probe of a modification to the embodiment.
FIG. 34 is an exemplary and schematic diagram illustrating the shielding member of FIG. 33 in which a rotational posture around a central axis is different. Description of Embodiments

Hereinafter, exemplary embodiments of the present invention and modifications thereto are disclosed. Configurations of the embodiments and the modifications described below, and functions and results (effects) provided by the configurations thereof are examples. The present invention can also be realized by configurations other than those disclosed in the following embodiments and modifications. In addition, according to the present invention, it is possible to obtain at least one of various effects (including derivative effects also) achieved by the configuration.

The plurality of embodiments and modifications described below have similar configurations. Therefore, according to the configurations of the embodiments and modifications, similar functions and effects based on the similar configurations can be obtained. In addition, in the following description, the similar configurations are given similar symbols, and duplicate descriptions may be omitted.

In the present specification, ordinal numbers are given for convenience in order to distinguish components, parts, directions, and the like, and do not indicate priority or order.

In the drawings, the X direction is an axial direction (longitudinal direction) of an irradiation probe 10.

### (First Embodiment)

### (Configuration of Irradiation Probe System)

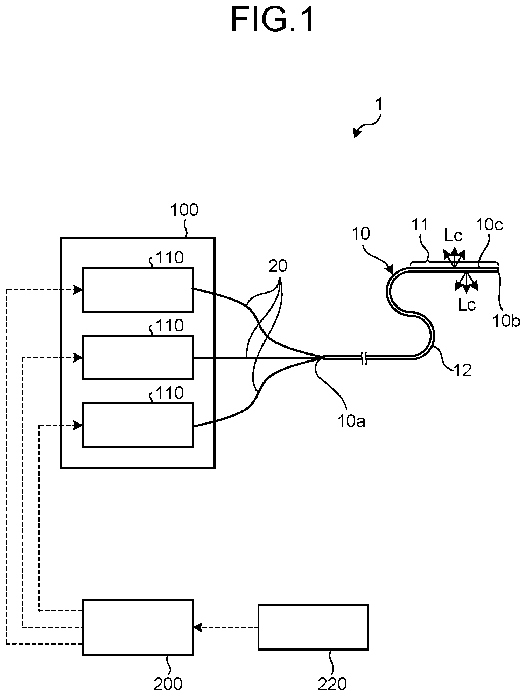

FIG. 1 is a schematic diagram of an irradiation probe system 1 according to an embodiment. As illustrated in FIG. 1, the irradiation probe system 1 includes a light output device 100, the irradiation probe 10, a control device 200, a delivery optical fiber 20, and an input unit 220.

The light output device 100 includes a plurality of light source units 110. Each of the light source units 110 includes a light source that outputs laser light and an optical system that guides light from the light source to the delivery optical fiber 20 (both are not illustrated). The light source includes, for example, a laser device that outputs laser light. Further, in the embodiment, the light output device 100 includes the plurality of light source units 110, that is, the light sources as an example, but the light output device 100 is not limited thereto. It is only required that the light output device 100 includes at least one light source unit 110.

Each of the light source units 110 and the irradiation probe 10 are optically connected via the delivery optical fiber 20 provided to correspond to the subject light source unit 110.

The irradiation probe 10 includes a plurality of optical fibers, has an elongated substantially cylindrical and linear shape, and has flexibility. The irradiation probe 10 has an end portion 10a that is one end in the axial direction and an end portion 10b that is the other end in the axial direction. The end portion 10a is an input end to which light from the light source units 110 is input, and may also be referred to as a base end. The end portion 10b is positioned on the side opposite to the end portion 10a in the axial direction, and may also be referred to as a distal end.

The irradiation probe 10 includes a leakage portion 11 and a transmission portion 12. The leakage portion 11 is provided over a predetermined length in the axial direction at a position away from the end portion 10a, and is a section that leaks light radially outward from an outer surface 10c of the irradiation probe 10. Light leaking from the outer surface 10c as a side surface of the leakage portion 11 is irradiation light from the irradiation probe 10. In addition, the transmission portion 12 is a section that transmits light between the end portion 10a and the leakage portion 11, between the leakage portion 11 and the end portion 10b, or, in a case where the plurality of leakage portions 11 is axially provided at an interval, the transmission portion 12 is a section that transmits light between the two leakage portions 11 sandwiching the interval. In the embodiment, as an example, the leakage portion 11 is provided only in the section adjacent to the end portion 10b, but the present invention is not limited thereto, and the leakage portion 11 may be provided apart from the end portion 10b.

The control device 200 can control the light source units 110 to output light or stop the output, for example. Further, the control device 200 can control operations of devices and portions other than the light source units 110 in the irradiation probe system 1. The input unit 220 constitutes a user interface operated by an operator (user), and inputs an instruction signal to the control device 200 in response to an operation input of the operator. The control device 200 is an example of a control mechanism, and the input unit 220 is an example of an operation input unit.

### (Configuration of Irradiation Probe)

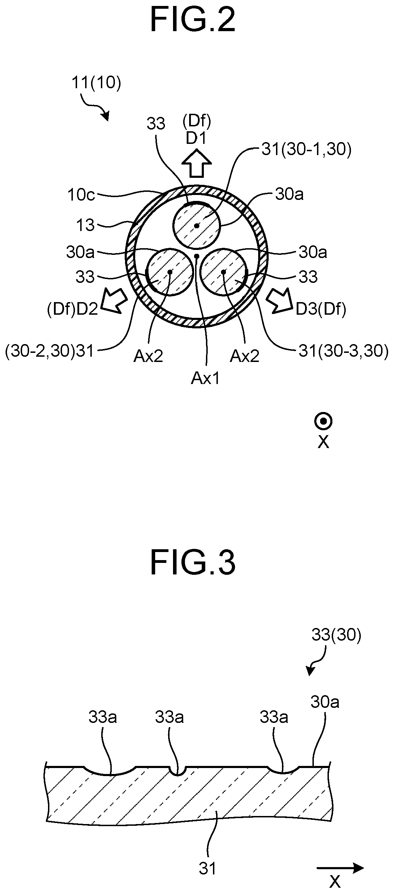

FIG. 2 is a cross-sectional view of the leakage portion 11 of the irradiation probe 10. As illustrated in FIG. 2, the irradiation probe 10 includes a plurality of optical fibers 30 bundled together and a cover 13 transparent to light transmitted through the optical fibers 30. In the embodiment, as an example, the irradiation probe 10 includes the three optical fibers 30, but the number of optical fibers 30 is not limited to three, and may be two or four or more.

The plurality of optical fibers 30 is arranged at substantially equal intervals and in a substantially rotationally symmetric manner around a central axis Ax1 of the irradiation probe 10. In other words, the optical fibers 30 are placed to be shifted from the central axis Ax1 of the irradiation probe 10 in different radial directions D1 to D3. Note that the irradiation probe 10 may include a holding member (not illustrated) that holds the plurality of optical fibers 30 in a predetermined relative positional relationship in a cross section intersecting the axial direction of the leakage portion 11.

The optical fibers 30 are optically connected to the delivery optical fibers 20, respectively. The optical fiber 30 and the delivery optical fiber 20 may be directly connected by fusion or the like, or indirectly connected via a coupling portion or the like, or the optical fiber 30 and the delivery optical fiber 20 may be made of a single optical fiber.

Each of the optical fibers 30 includes a core 31 and a clad (not illustrated) surrounding the core 31. In the transmission portion 12, the optical fiber 30 includes the core 31 and the clad. On the other hand, in the leakage portion 11, for example, as illustrated in FIG. 2, the clad is substantially removed from each optical fiber 30, and only the cores 31 are bundled together. That is, in the example of FIG. 2, an outer surface 30a of each optical fiber 30 is an outer surface of the core 31.

In the leakage portion 11, a scattering region 33 in which light is scattered is provided in at least one of the outer surface 30a and a range having a predetermined depth in the vicinity of the outer surface 30a. The scattering region 33 extends in the circumferential direction. Specifically, as illustrated in FIG. 2, the scattering region 33 is provided in a partial section in the circumferential direction, specifically, in a fan-shaped arc having a predetermined central angle (In FIG. 2, as an example, 60 deg.) or in a range near the fan-shaped arc, in a cross section orthogonal to a central axis Ax2 of the optical fiber 30. Although not illustrated, the scattering region 33 extends also in the axial direction (longitudinal direction). In other words, the scattering region 33 is provided over a predetermined section in the longitudinal direction of the leakage portion 11. The scattering region 33 may be provided over the entire region of the leakage portion 11, may be provided over a part of the region of the leakage portion 11, or may be intermittently provided at a plurality of positions of the leakage portion 11. In a case where a plurality of scattering regions 33 is provided in the leakage portion 11, the scattering regions 33 are provided side by side in the longitudinal direction. In the optical fiber 30, the section in which the scattering region 33 is provided is an example of a leakage section. The leakage section is included in the leakage portion 11. In other words, the leakage section of the optical fiber 30 in which the scattering region 33 is provided is a part of the constituent elements of the leakage portion 11 of the irradiation probe 10.

FIG. 3 is a cross-sectional view of the optical fiber 30 at a portion where the scattering region 33 is provided. As illustrated in FIG. 3, in the scattering region 33, a plurality of recesses 33a is formed on the outer surface 30a. Light transmitted in the core 31 is refracted and scattered in the recesses 33a, and leaks from the outer surface 30a to the outside of the core 31, that is, the outside of the optical fiber 30.

In the example of FIG. 3, the recesses 33a are discretely formed, and the size and depth of the recesses 33a are not constant. However, this is an example, and the plurality of recesses 33a may be regularly arranged, or specifications of the plurality of recesses 33a, e.g., a size, a depth, and a shape may be substantially constant. In the scattering region 33, instead of the recess 33a, a protrusion may be formed on the outer surface 30a. The protrusion may be, for example, a portion between the recess 33a and the recess 33a. In the embodiment, leakage of light radially outward from the core 31 is facilitated due to the recesses 33a and the protrusions. The specifications of the recesses 33a and the protrusions, e.g., locations, densities, the size, and the depth thereof, are appropriately adjusted, so that the distribution of the intensity of light leakage in the axial direction and the circumferential direction of the leakage portion 11 can be appropriately adjusted.

The scattering region 33 is appropriately provided in the optical fiber 30. As a result, the optical fiber 30 can be configured as an optical fiber in which the intensity of leakage light to a specific radial direction (radially outward) from the central axis Ax2 of the optical fiber 30 is higher than the intensity of leakage light to the other radial directions in the intensity distribution in the circumferential direction of the leakage light in the cross section intersecting the axial direction, that is, the optical fiber 30 can be configured as an optical fiber having directivity. In the example of FIG. 2, in each optical fiber 30, the intensity of the leakage light to a radial direction Df is higher than the intensity thereof in the other radial directions. The radial direction Df is an example of the specific radial direction. In the present specification, the specific radial direction is a radial direction where the peak is reached in the intensity distribution in the circumferential direction of the optical fiber 30 of the leakage light from the optical fiber 30. In a case where there is a plurality of radial directions (for example, two directions) where the peak is reached, each direction is the specific radial direction.

Here, as described above, in the embodiment, the optical fibers 30 having the above-described directivity of the leakage light are disposed apart from the central axis Ax1 of the irradiation probe 10 in the radial directions D1 to D3 different from each other. The optical fibers 30 are bundled together in a posture in which the leakage light to the radial direction Df from the scattering region 33 is directed radially outward of the irradiation probe 10 in the leakage portion 11. In the embodiment, as an example, as illustrated in FIG. 2, each of the optical fibers 30 is arranged in a posture in which the scattering region 33 is located farthest from the central axis Ax1 of the irradiation probe 10, so that leakage light output to the radial direction Df from the optical fibers 30 is output toward the radial directions D1 to D3 (radially outward) different from each other in the irradiation probe 10.

The light source units 110 are optically connected to the optical fibers 30 different from each other. Therefore, the control device 200 controls the light output device 100 so that any one of the plurality of light source units 110 selectively outputs light, to thereby select, from among the plurality of optical fibers 30, the optical fiber 30 that outputs leakage light. Here, as described above, leakage light is output from the leakage portion 11 of the irradiation probe 10 to the radial directions D1 to D3 different from each other according to the optical fibers 30. Therefore, the control device 200 selectively operates the light source units 110 to thereby switch the output direction of leakage light from the leakage portion 11, that is, irradiation light output from the irradiation probe 10, namely, the radial directions D1 to D3. The control device 200 is an example of a switching mechanism.

### (Control over Irradiation System)

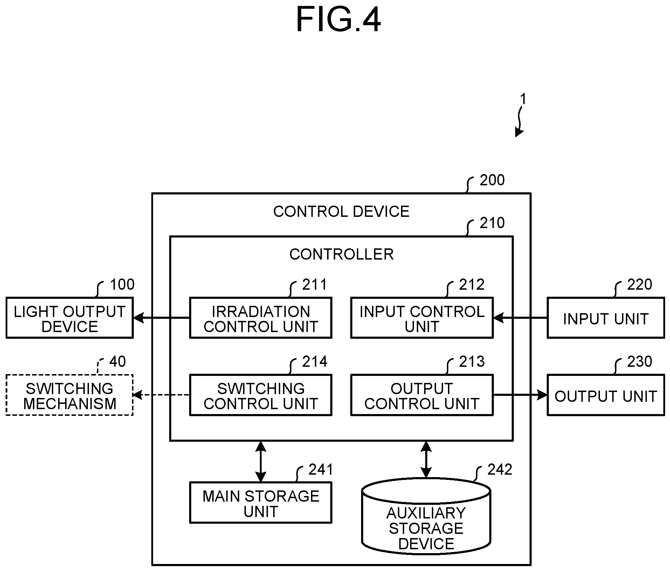

FIG. 4 is a block diagram of the irradiation probe system 1. As illustrated in FIG. 4, the irradiation probe system 1 includes the control device 200, the input unit 220, and an output unit 230. The input unit 220 and the output unit 230 construct a user interface for the user and the operator. The input unit 220 is, for example, an input device such as an operation unit, e.g., a remote controller, a switch box, or a joystick, or an input device, e.g., a keyboard, a touch panel, a mouse, a switch, or an operation button. The output unit 230 is, for example, a display, a printer, a lamp, a speaker, or the like, and is an output device using image, printing, or audio.

The control device 200 includes a controller 210, a main storage unit 241, and an auxiliary storage device 242.

The controller 210 is, for example, a processor (circuit) such as a central processing unit (CPU). The main storage unit 241 is, for example, a random access memory (RAM) or a read only memory (ROM). The auxiliary storage device 242 is a nonvolatile rewritable storage device such as a solid state drive (SSD) or a hard disk drive (HDD).

The controller 210 operates as an irradiation control unit 211, an input control unit 212, and an output control unit 213 by reading programs stored in the main storage unit 241 and the auxiliary storage device 242 to execute each processing. Each of the programs may be provided by being recorded on a computer-readable recording medium in an installable or executable format file. The recording medium may also be referred to as a program product. Information such as values, maps, and tables used in arithmetic processing by the programs and the processor may be stored in advance in the main storage unit 241 or the auxiliary storage device 242, or may be stored in a storage unit of a computer connected to a communication network and stored in the auxiliary storage device 242 by being downloaded via the communication network. The auxiliary storage device 242 stores data written by the processor. Further, the arithmetic processing by the controller 210 may be executed at least partially by hardware. In this case, the controller 210 may include, for example, a field programmable gate array (FPGA), an application specific integrated circuit (ASIC), or the like.

The irradiation control unit 211 can control each of the light source units 110 included in the light output device 100 to output light and stop outputting the light. Further, the irradiation control unit 211 can switch the light source unit 110 that outputs light, among the plurality of light source units 110 (light sources), in response to the operator inputting operation to the input unit 220. In other words, the irradiation control unit 211 operates to switch the irradiation directions (radial directions D1 to D3) of light from the irradiation probe 10.

The input control unit 212 receives an input signal from the input unit 220. Further, the input control unit 212 may control the input unit 220 to enable predetermined operation input.

The output control unit 213 controls the output unit 230 to execute predetermined output.

### (Example of Irradiation Control)

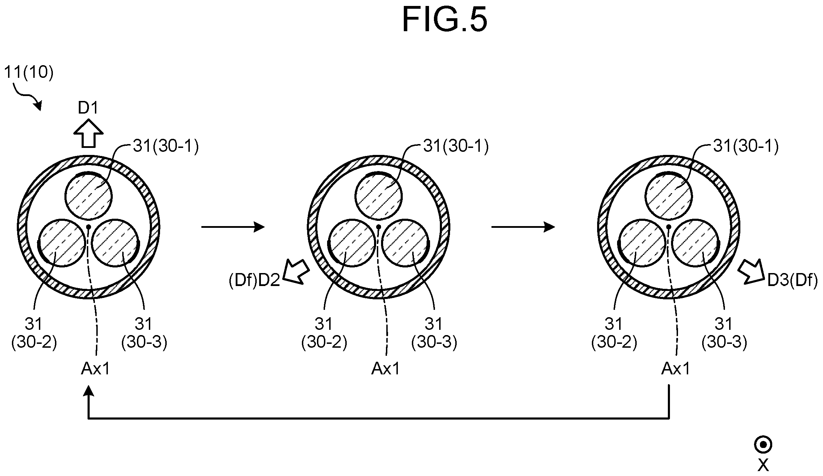

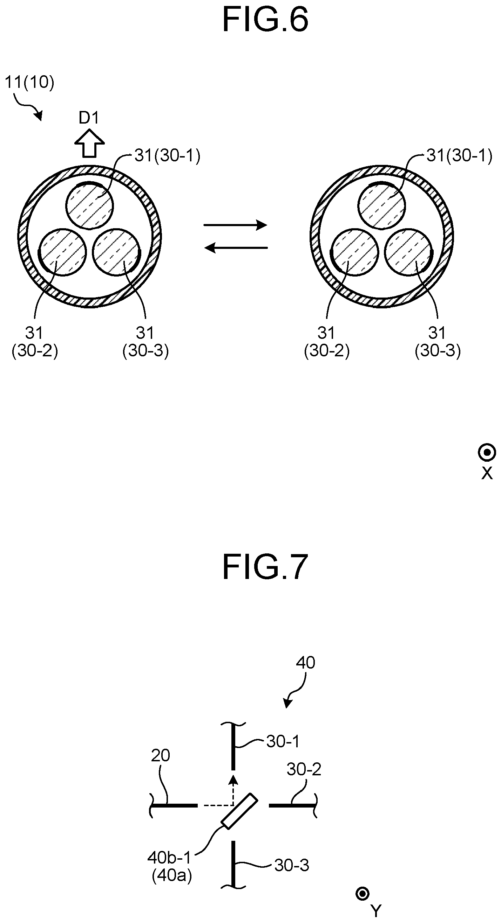

FIGS. 5 and 6 are cross-sectional views of the leakage portion 11 illustrating an example of a temporal change in a light irradiation state by the irradiation probe 10.

In the example of FIG. 5, the irradiation control unit 211 sequentially switches between the operation state and the operation stop state of the three light source units 110, so that a state in which light is input from the light output device 100 to the optical fiber 30-1, a state in which light is input from the light output device 100 to the optical fiber 30-2, and a state in which light is input from the light output device 100 to the optical fiber 30-3 are sequentially and repeatedly switched. Thereby, a state in which the irradiation light as the leakage light is output from the leakage portion 11 in the radial direction D1 (upward in FIG. 5), a state in which the irradiation light as the leakage light is output in the radial direction D2 (lower left in FIG. 5), and a state in which the irradiation light as the leakage light is output in the radial direction D3 (lower right in FIG. 5) are sequentially and repeatedly switched. In this case, the irradiation light from the irradiation probe 10 rotates around the central axis Ax1 of the irradiation probe 10.

In the example of FIG. 6, the irradiation control unit 211 switches between the operation state and the operation stop state of one light source unit 110, so that a state in which light is input from the light output device 100 to the optical fiber 30-1 and a state in which light is not input to any of the optical fibers 30 are alternately switched. As a result, the irradiation light as the leakage light is intermittently output in the radial direction D1 (upward in FIG. 6). In other words, the irradiation light output in the radial direction D1 flashes at predetermined time intervals.

The intensity of light irradiation can be reduced and the damage to the living tissue due to the light and heat can be reduced by the rotation of irradiation light as illustrated in FIG. 5 or the flash of irradiation light as illustrated in FIG. 6. The rotation direction, the rotation speed, the flash speed, changes thereof, and the like are not limited to the examples of FIGS. 5 and 6, and can be appropriately set.

As described above, in the embodiment, the control device 200 operates, so that the irradiation direction of light (leakage light and irradiation light) from the irradiation probe 10 can be switched without changing the rotational posture around the central axis Ax1 of the irradiation probe 10. Therefore, for example, in a case where the irradiation direction is changed because light is applied, from the irradiation probe 10, to a region different from a region originally intended to be irradiated with light, or because light is to be applied in different radial directions, changing the rotational posture of the irradiation probe 10 is unnecessary or minimized, so that the irradiation direction can be changed more easily or more quickly.

Further, in the embodiment, the control device 200 operates to rotate light around the central axis Ax1 of the irradiation probe 10 or flash light, so that the irradiation intensity, the irradiation area, and the irradiation timing of the light are adjusted, and a more appropriate light irradiation state for the affected area can be easily achieved.

### (Second Embodiment)

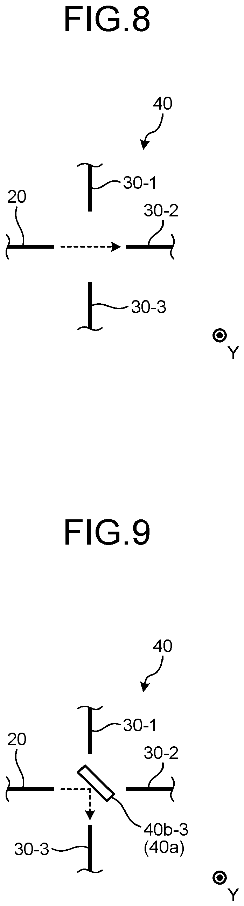

FIGS. 7 to 9 are schematic diagrams of a switching mechanism 40 included in the irradiation probe system 1 according to the second embodiment. FIG. 7 illustrates a state in which light from the light output device 100 is input to the optical fiber 30-1, FIG. 8 illustrates a state in which light from the light output device 100 is input to the optical fiber 30-2, and FIG. 9 illustrates a state in which light from the light output device 100 is input to the optical fiber 30-3. In the irradiation probe system 1 of the embodiment, the switching mechanism 40 can select the optical fiber 30 that inputs light from the delivery optical fiber 20, that is, the optical fiber 30 that leaks and outputs light in the leakage portion 11.

The switching mechanism 40 includes, for example, a movable portion 40a movable in the Y direction in FIG. 7 to 9, and the movable portion 40a includes mirrors 40b-1 and 40b-3 provided at different positions in the Y direction. The switching mechanism 40 also includes a drive mechanism (not illustrated) capable of changing the position of the movable portion 40a in the Y direction. The drive mechanism includes, for example, a motor, a speed reduction mechanism that decelerates rotation of the motor, and a motion conversion mechanism that converts rotation of the speed reduction mechanism into linear motion of the movable portion 40a along the Y direction. The operation of the switching mechanism 40 (drive mechanism) is controlled by a switching control unit 214 included in the controller 210 as illustrated in FIG. 4. Note that the drive mechanism is not limited to have such a configuration, and may include another mechanism such as an electromagnetic solenoid.

In such a configuration, the switching mechanism 40 changes the position of the movable portion 40a in the Y direction, so that the switching mechanism 40 can switch a state in which light from the light output device 100 is reflected by the mirror 40b-1 and then coupled to the optical fiber 30-1 (FIG. 7), a state in which light from the light output device 100 is coupled to the optical fiber 30-2 without passing through the mirrors 40b-1 and 40b-3 (FIG. 8), and a state in which light from the light output device 100 is reflected by the mirror 40b-3 and then coupled to the optical fiber 30-3 (FIG. 9).

The configuration of the irradiation probe 10 is similar to that of the first embodiment. Therefore, also in the embodiment, it is possible to selectively output light from any one of the plurality of optical fibers 30 in the leakage portion 11, and thus, it is possible to obtain the functions and effects similar to those of the first embodiment.

Then, according to the embodiment, it is not necessary to provide one light source unit 110 for each of the optical fibers 30, and accordingly, the number of light source units 110 in the light output device 100 can be further reduced. As a result, it is possible to obtain advantages that the light output device 100 can be configured to be smaller or lighter and that the labor and cost involved in manufacturing the light output device 100 can be reduced.

### (Configuration Example (Modification) of Scattering Region)

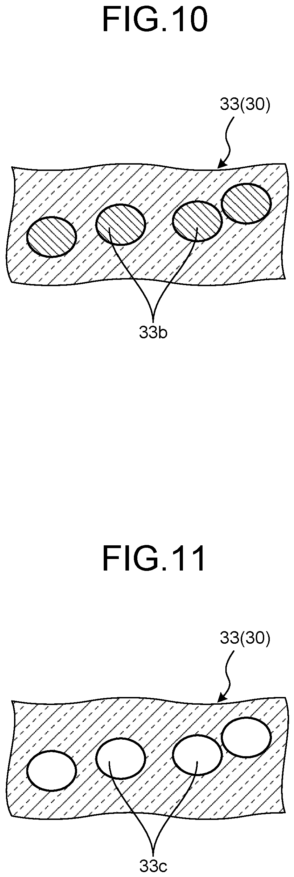

FIGS. 10 and 11 are cross-sectional views each illustrating an example of a configuration of the scattering region 33. In the example of FIG. 10, particles 33b are included inside the optical fiber 30 in which the scattering region 33 is formed, and in the example of FIG. 11, holes 33c are included inside the optical fiber 30 in which the scattering region 33 is formed. The particles 33b and the holes 33c may have, for example, a nanostructure with a diameter of 100 [nm] or less. The particles 33b may be, for example, fillers such as fine particles or fine tubes. In these cases, the direction of light travel is changed due to the particles 33b and the holes 33c, that is, light is scattered. Thus, light easily leaks radially outward from the outer surface 30a.

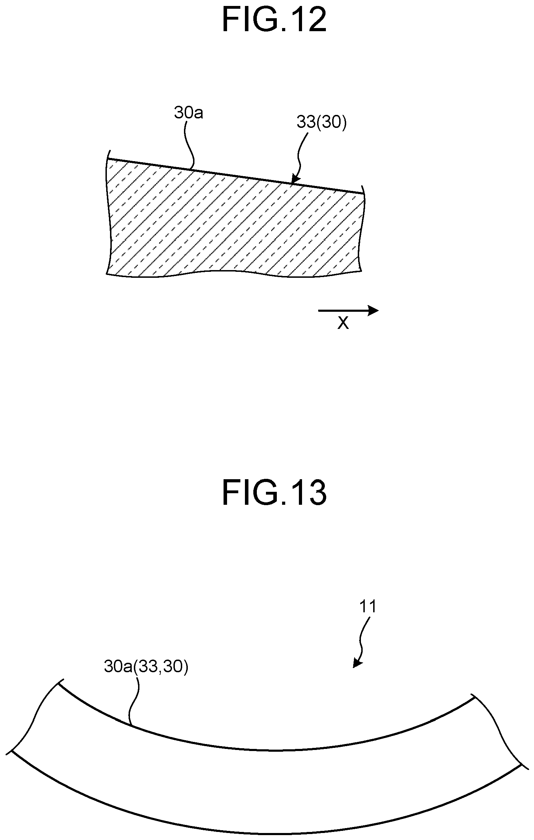

FIG. 12 is a cross-sectional view illustrating another example of the configuration of the scattering region 33. In the example of FIG. 12, the outer surface 30a of the optical fiber 30 is inclined to the X direction that is the longitudinal direction of the optical fiber 30. The outer surface 30a is, for example, a tapered surface or the like. As described above, in a part where the shape of the outer surface 30a changes as the outer surface 30a extends in the X direction, for example, light enters the part beyond the critical angle, so that the light easily leaks radially outward from the outer surface 30a. The particles 33b and the holes 33c may also be referred to as scattering elements.

FIG. 13 is a side view illustrating another example of the configuration of the scattering region 33. In the example of FIG. 13, the scattering region 33 is curved. Light easily leaks from the curve part. That is, also with the configuration of FIG. 13, light easily leaks radially outward from the outer surface 30a.

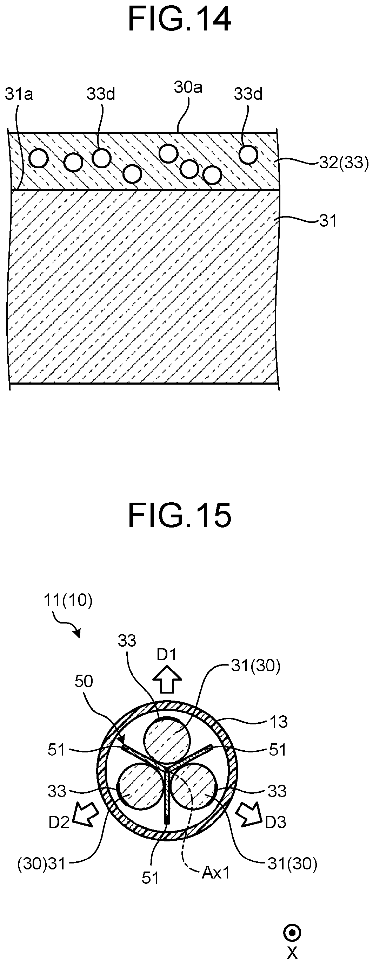

FIG. 14 is a cross-sectional view illustrating another example of the configuration of the scattering region 33. In the example of FIG. 14, the scattering region 33 includes a cover layer 32 that at least partially covers an outer surface 31a of the core 31. The refractive index of the cover layer 32 is set to be substantially the same as or higher than the refractive index of the core 31. Further, the cover layer 32 includes scattering elements 33d such as particles or holes. In this case, light that has reached the interface between the core 31 and the cover layer 32 enters the cover layer 32, is scattered by the scattering element 33d, and leaks radially outward. According to such a configuration, for example, it is possible to obtain an advantage that the provision of the cover layer 32 enables appropriately setting or changing a place where light leaks, a place where light easily leaks, or a place where the intensity of leakage light becomes high. In addition, in a case where the core 31 is appropriately pressurized radially inward by the cover layer 32, light easily leaks from the pressurized part.

The configurations illustrated in FIGS. 10 to 14 may be appropriately combined in the optical fiber 30 for implementation.

### (Shielding Member (Modification) 1: Plate)

FIG. 15 is a cross-sectional view illustrating an example of a configuration of the leakage portion 11. In the example of FIG. 15, the leakage portion 11 includes a shielding member 50. The shielding member 50 blocks leakage light from the optical fibers 30 positioned to be shifted from the central axis Ax1 of the irradiation probe 10 in the radial directions D1 to D3 (hereinafter, referred to as eccentric directions) from being directed in the direction opposite to the eccentric direction and the circumferential direction of the irradiation probe 10.

The specifications of the scattering region 33 are adjusted, so that leakage light having high directivity in the radial directions D1 to D3, which are eccentric directions of the optical fibers 30, is output from each optical fiber 30. However, although the ratio is low, a part of the leakage light is output in a direction other than the radial directions D1 to D3, that is, in a direction different from the intended direction. Such light output in a direction different from the intended direction causes a decrease in directivity as the irradiation probe 10, and the light is unnecessarily applied to, for example, even a region in the living body where the light is not required to be applied originally or is not to be applied. In this regard, according to the modification of FIG. 15, the shielding member 50 can prevent leakage light (irradiation light) from the irradiation probe 10 from being output in a direction other than the intended directions (radial directions D1 to D3), and it is thus possible to prevent a decrease in directivity as the irradiation probe 10, and it is also possible to prevent the light from being unnecessarily applied to, for example, even a region in the living body where the light is not required to be applied originally or is not to be applied.

In the example of FIG. 15, the shielding member 50 includes a plurality of plates 51. Each of the plates 51 extends in a radial direction from the central axis Ax1 so as to pass between the two optical fibers 30. In other words, the plate 51 is positioned between the two optical fibers 30 in the circumferential direction of the irradiation probe 10. The plurality of plates 51 is connected to each other and integrated on or near the central axis Ax1, that is, radially inward of the irradiation probe 10. In such a configuration, for each of the optical fibers 30, two plates 51 are located in the opposite direction to the eccentric direction of the subject optical fiber 30, and are located on both sides in the circumferential direction of the subject optical fiber 30. Such a configuration prevents leakage light from the optical fibers 30 from traveling in the opposite direction to the eccentric direction and the circumferential direction of the irradiation probe 10. The plate 51 is an example of an intervening portion and an example of an extending portion.

The shielding member 50 includes, for example, a metal member, and shields leakage light from the optical fiber 30. The shielding member 50 may be entirely made of a metal material such as, for example, a copper-based material. This enhances heat dissipation in the leakage portion 11 and reduces the rise in temperature of the leakage portion 11. In this case, the shielding member 50 is an example of the metal member. Further, the shielding member 50 may include, for example, a core member having an elastic modulus smaller than that of a metal material such as a synthetic resin material, and a cover made of a metal material covering a surface of the core member. In this case, the shielding member 50 and thus the leakage portion 11 can be configured more flexibly. In this case, the cover is an example of the metal member.

The shielding member 50 may reflect leakage light. The shielding member 50 reflects leakage light traveling in a direction different from the intended direction in a direction close to the intended direction, which improves the directivity as the irradiation probe 10. In this case, the shielding member 50 is an example of a reflective member.

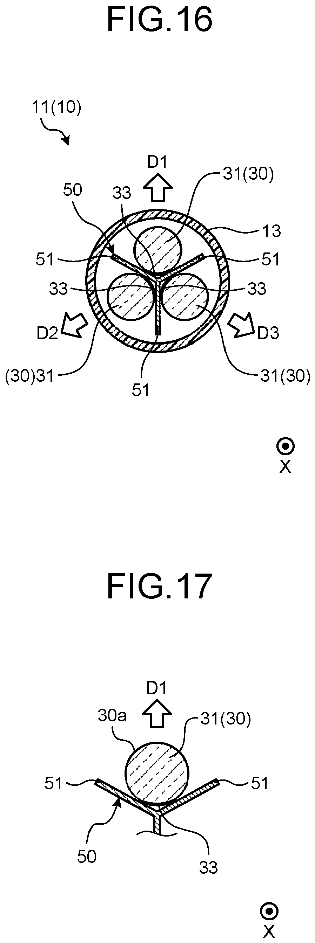

FIG. 16 is a cross-sectional view illustrating an example of the configuration of the leakage portion 11. In the example of FIG. 15, the scattering region 33 is located radially outward of the irradiation probe 10 in each optical fiber 30, whereas in the example of FIG. 16, the scattering region 33 is located radially inward of the irradiation probe 10 in each optical fiber 30. The scattering region 33 in the case of FIG. 15 is an example of an inner scattering portion, and the scattering region 33 in the case of FIG. 16 is an example of an outer scattering portion.

In addition, each of the optical fibers 30 has directivity in which the intensity of leakage light directed radially outward of the irradiation probe 10 and the intensity of leakage light directed radially inward of the irradiation probe 10 in the posture of FIG. 16 are higher than the intensity of leakage light in the other directions, in the intensity distribution in the circumferential direction of the optical fiber 30 of leakage light by adjusting the specifications of the scattering region 33. In other words, the optical fibers 30 have radiation characteristics of leakage light having high directivity in two directions of the radial directions D1 to D3 (eccentric directions, radially outward) and a direction opposite to the eccentric directions (radially inward) in the posture of bundled optical fibers 30 as illustrated in FIG. 16. Further, the shielding member 50 is an example of the reflective member, and is configured to reflect leakage light.

In this case, for each optical fiber 30, part of the light (scattered light) directed radially outward of the irradiation probe 10 in the scattering region 33 is totally reflected by a surface (hereinafter, referred to as a facing surface) on the opposite side of the scattering region 33 of the optical fiber 30 and remains in the optical fiber 30. Further, another part of the light directed radially outward of the irradiation probe 10 in the scattering region 33 does not satisfy total reflection conditions on the facing surface, and thus, leaks from the facing surface to outside of the subject optical fiber 30, and is directed in the radial directions D1 to D3 or a direction close to the radial directions D1 to D3.

On the other hand, part of the light (scattered light) directed radially inward of the irradiation probe 10 in the scattering region 33 is reflected by the shielding member 50 and then directed in the radial directions D1 to D3 or a direction close to the radial directions D1 to D3. Further, another part of the scattered light directed radially inward of the irradiation probe 10 in the scattering region 33 enters the optical fiber 30 again, and is divided, at the facing surface, into light remaining in the optical fiber 30 and light to be output from the facing surface in the radial directions D1 to D3 or in a direction close to the radial directions D1 to D3.

With such a configuration, the leakage portion 11 outputs leakage light having high directivity in the eccentric directions (radial directions D1 to D3) of the optical fibers 30.

In this example, most of the scattered light from the scattering region 33, including the light that is reflected by the shielding member 50 to enter again the optical fiber 30, is output to the outside of the leakage portion 11 through the facing surface. On the facing surface, light that does not satisfy the total reflection conditions, that is, light having a small inclination angle with respect to the radial directions D1 to D3, selectively leaks to the outside of the optical fiber 30. Therefore, according to the configuration in which the scattering region 33 is located radially inward of the irradiation probe 10 in each optical fiber 30 as in the examples of FIGS. 16 and 17, the directivity in the radial directions D1 to D3 as the irradiation probe 10 is increased as compared with the configuration in which the scattering region 33 is located radially inward of the irradiation probe 10 in each optical fiber 30.

FIG. 17 is a partially enlarged view of a modification to FIG. 16. In the example of FIG. 17, the average radius of curvature in the scattering region 33 is the same as a radius of a general region of the outer surface 30a of the optical fiber 30 in which the scattering region 33 is not formed. In this case, in the scattering region 33, the outer surface 30a is a convex curved surface.

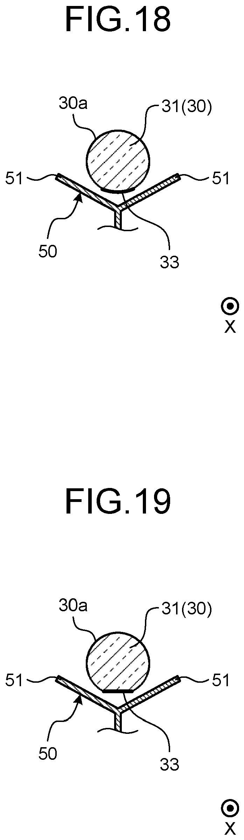

FIG. 18 illustrates another modification in which the shape of the scattering region 33 is changed from the example of FIG. 17. In the example of FIG. 18, the average radius of curvature of the outer surface 30a in the scattering region 33 is equal to or greater than a radius of a general region of the outer surface 30a of the optical fiber 30 in which the scattering region 33 is not formed. Also in this case, in the scattering region 33, the outer surface 30a is a convex curved surface.

FIG. 19 illustrates another modification in which the shape of the scattering region 33 is changed from the example of FIG. 17. In the example of FIG. 19, the outer surface 30a in the scattering region 33 is a plane intersecting the radial direction of the optical fiber 30.

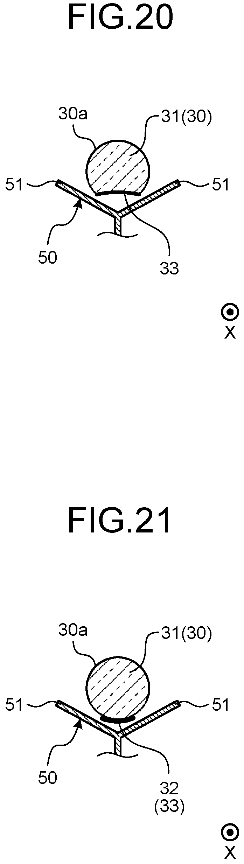

FIG. 20 illustrates another modification in which the shape of the scattering region 33 is changed from the example of FIG. 17. In the example of FIG. 20, the outer surface 30a in the scattering region 33 is a concave curved surface that is concave radially inward of the optical fiber 30.

The inventors have intensively studied various modifications as illustrated in FIGS. 17 to 20, and found that a distribution ratio between scattered light (hereinafter, referred to as first scattered light) traveling from the scattering region 33 to radially inward of the irradiation probe 10, that is, traveling in a direction opposite to the eccentric direction of each optical fiber 30, and scattered light (hereinafter, referred to as second scattered light) traveling from the scattering region 33 to radially outward of the irradiation probe 10, that is, traveling in the eccentric direction (radial directions D1 to D3) of each optical fiber 30 can be adjusted according to the specifications of the scattering region 33. Hereinafter, the ratio of the second scattered light to the first scattered light is referred to as a branching ratio.

Specifically, it has been found that, in a case where the average radius of curvature of the outer surface 30a in the scattering region 33 is greater than the radius of the outer surface 30a in the general region as illustrated in FIG. 18, the branching ratio can be increased as compared with a case where the average radius of curvature of the outer surface 30a in the scattering region 33 is the same as the radius of the general region as illustrated in FIG. 17. Further, it has been found that, in a case where the outer surface 30a in the scattering region 33 is a flat surface or a concave curved surface as illustrated in FIGS. 19 and 20, the branching ratio can be reduced as compared with a case where the radius of curvature of the outer surface 30a in the scattering region 33 is the same as the radius of the general region. Further, it has been found that the magnitude of the branching ratio can be adjusted by adjusting various specifications of the scattering region 33 such as the radius of curvature and the circumferential length of the scattering region 33. As a result, for example, an effect of increasing the degree of freedom of design as the irradiation probe 10 can be obtained.

In the examples of FIGS. 17 to 20, the scattering element in the scattering region 33 can be configured as the recess 33a (see FIG. 3) or the protrusion formed on the outer surface 30a, the particles 33b (see FIG. 10) or the holes 33c (see FIG. 11) provided inside and in the vicinity of the outer surface 30a, or the like.

Further, in forming the recesses 33a or the protrusions in the scattering region 33, masking is performed on the outer surface 30a of the optical fiber 30 except for a part where the scattering region 33 is to be formed, and processing of forming an uneven surface such as sandblasting is performed on an unmasked opening part, whereby the recesses 33a or the protrusions can be formed. In this case, the shape and the radius of curvature of the scattering region 33 can be appropriately adjusted by performing masking in multiple stages or adjusting the irradiation time according to the irradiation direction for sandblasting.

FIG. 21 illustrates another modification in which the shape of the scattering region 33 is changed from the example of FIG. 17. In FIG. 21, the scattering region 33 is formed as the cover layer 32 as illustrated in FIG. 14. The cover layer 32 can be configured as a part of a thin cladding layer. Also in such a configuration, functions and effects similar to those in the examples of FIGS. 15 to 20 can be obtained.

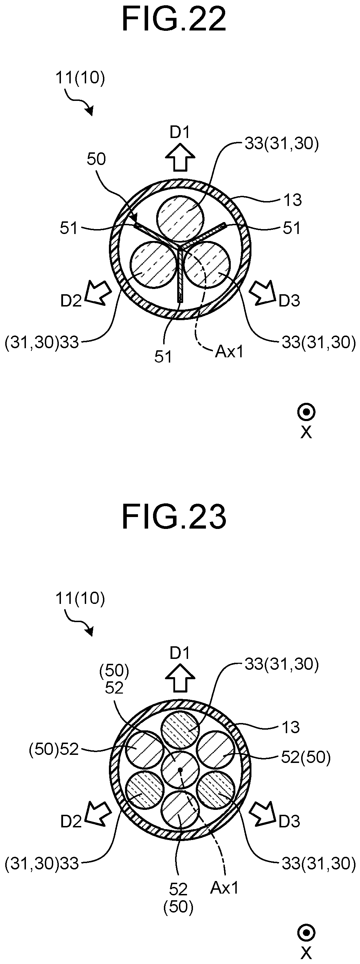

FIG. 22 is a cross-sectional view of a modification in which the directivity of the leakage light by the optical fiber 30 is reduced as compared with the examples of FIGS. 15 and 16. Instead of having the scattering region 33 only in a specific part in the circumferential direction, in the example of FIG. 22, the optical fiber 30 has the scattering element substantially uniformly or entirely in the substantially entire region of the cross section or the outer surface 30a or the region close to the outer surface 30a. In this case, the intensity distribution of the leakage light in the circumferential direction of the optical fiber 30 is a relatively flat distribution without a peak in a specific radial direction. However, also in this case, by configuring the shielding member 50 as a reflective member, leakage light (irradiation light) having relatively high directivity in the eccentric directions (radial directions D1 to D3) of the optical fiber 30 is output from the irradiation probe 10 including the reflected light at the shielding member 50.

In the examples of FIGS. 15 and 16, the plurality of plates 51 may be separated from each other. In this case, the boundary between the plurality of plates 51 may be covered with another shielding member (not illustrated).

### (Shielding Member (Modification) 2: Dummy Fiber)

FIG. 23 is a cross-sectional view illustrating an example of the configuration of the leakage portion 11. In the example of FIG. 23, the shielding member 50 includes a plurality of dummy fibers 52. The plurality of dummy fibers 52 include the dummy fiber 52 located at the center of the cross section of the irradiation probe 10 and the dummy fiber 52 located between the two optical fibers 30. The optical fibers 30 and the dummy fibers 52 are alternately arranged in the circumferential direction around the dummy fiber 52 located at the center of the cross section.

The dummy fiber 52 includes, for example, a metal member, and shields leakage light from the optical fiber 30. In addition, for each optical fiber 30, the dummy fibers 52 are placed radially inward of the irradiation probe 10 and on both sides of the subject optical fiber 30 in the circumferential direction. Therefore, according to the example of FIG. 23, the plurality of dummy fibers 52 can function as the shielding members 50. The dummy fiber 52 is an example of an opaque fiber that does not transmit leakage light, and is an example of a first opaque fiber.

The dummy fiber 52 may be made of, for example, a conductive metal material such as a copper-based material. In this case, the dummy fiber 52 can be used as a conductor for power or an electric signal. Further, in this case, the dummy fiber 52 may have an insulating cover.

In the example of FIG. 23, the dummy fiber 52 may be a reflective member that reflects leakage light from the optical fiber 30. For example, the dummy fiber 52 may be made of a synthetic resin material having high reflectivity such as polytetrafluoroethylene (PTFE).

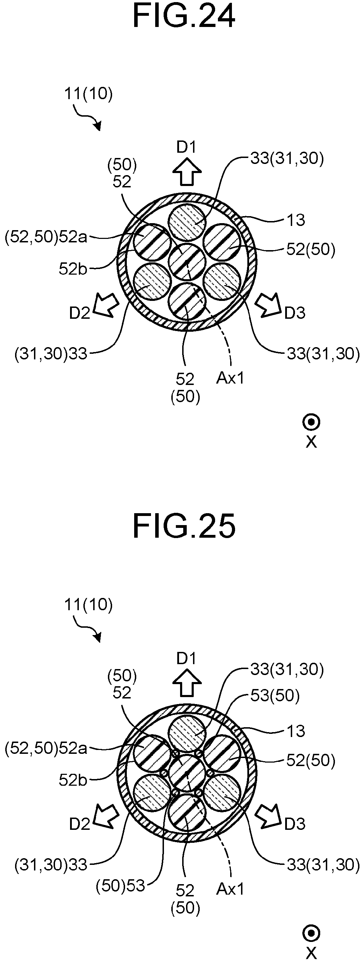

FIG. 24 is a cross-sectional view illustrating an example of the configuration of the leakage portion 11. In the example of FIG. 23, the dummy fiber 52 is entirely made of the metal member, whereas in the example of FIG. 24, the dummy fiber 52 includes a core member 52a having an elastic modulus smaller than that of a metal material such as a synthetic resin material, and a cover 52b made of a metal material covering the surface of the core member. In this case, the shielding member 50 and thus the leakage portion 11 can be configured more flexibly. In this case, the cover 52b is an example of the metal member.

FIG. 25 is a cross-sectional view illustrating an example of the configuration of the leakage portion 11. In the example of FIG. 25, a dummy fiber 53 is added as the shielding member 50 to the example of FIG. 24. The dummy fiber 53 includes, for example, a metal member, and shields leakage light from the optical fiber 30. In addition, the dummy fiber 53 has the same diameter as or a different diameter from the dummy fiber 52, is thinner than the dummy fiber 52 as an example, is located on a side closer to the optical fiber 30 with respect to a boundary between two dummy fibers 52 adjacent to each other, and covers the boundary with respect to the optical fiber 30. The dummy fiber 53 is an example of the opaque fiber, and is an example of a second opaque fiber.

Such a configuration prevents leakage light from the optical fiber 30 from traveling in a direction different from the intended direction (radial directions D1 to D3) via the boundary between the two dummy fibers 52.

Also in the example of FIG. 25, the dummy fiber 52 may be the reflective member that reflects leakage light from the optical fiber 30. The dummy fiber 52 may be the dummy fiber 52 without the core member 52a and the cover 52b as in the example of FIG. 23.

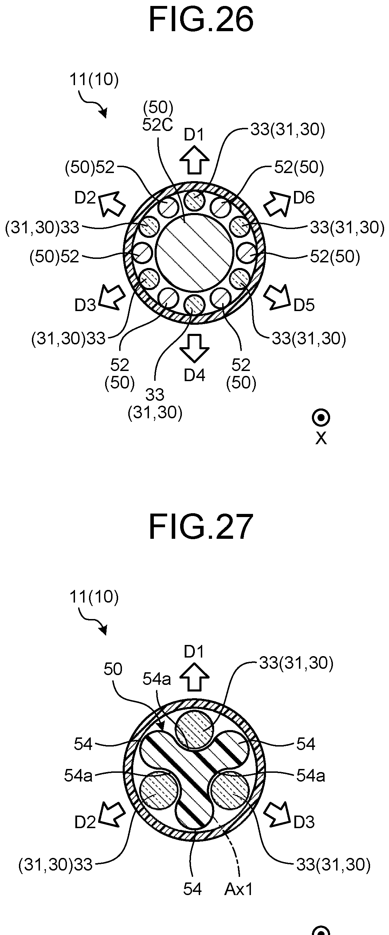

FIG. 26 is a cross-sectional view illustrating an example of the configuration of the leakage portion 11. In the example of FIG. 26, the leakage portion 11 has a configuration similar to that of the example of FIG. 23. However, the number of optical fibers 30 is six, which is greater than that of the example of FIG. 23. Further, the shielding member 50 includes a dummy fiber 52C located at the center of the cross section and the dummy fibers 52 alternately arranged on the outer periphery of the dummy fiber 52C together with the optical fibers 30. In addition, the diameter of the dummy fiber 52C is larger than the diameter of each of the optical fibers 30 and the dummy fibers 52, and the diameter of the dummy fibers 52 is substantially the same as the diameter of the optical fibers 30. The dummy fiber 52C is an example of a core wire extending in the longitudinal direction of the irradiation probe 10.

In this case, the dummy fiber 52C located at the center of the cross section can function as a support member of the plurality of optical fibers 30 and the dummy fibers 52 arranged on the outer periphery of the dummy fiber 52C or a guide at the time of manufacturing. Since the diameter of the dummy fiber 52C is larger than the diameter of the optical fibers 30, leakage light from each optical fiber 30 toward the direction opposite to the radial directions D1 to D6 can be more reliably blocked.

In the example of FIG. 26, the dummy fiber 52C and the dummy fibers 52 may be the reflective members that reflect leakage light from the optical fiber 30.

### (Shielding Member (Modification) 3: Others)

FIG. 27 is a cross-sectional view illustrating an example of the configuration of the leakage portion 11. In the example of FIG. 27, the shielding member 50 is formed with a recess 54a that accommodates the optical fiber 30 at least partially. The recess 54a can be formed, for example, by pressing the optical fiber 30 or a member different from the optical fiber 30 radially inward at the time of manufacturing. Alternatively, the shielding member 50 including the recess 54a may be molded by extrusion molding or the like. In the example of FIG. 27, at least a part of the shielding member 50 may be made of a relatively flexible material having an elastic modulus smaller than that of a metal material such as a synthetic resin material.

Protrusions 54 are provided on both sides of the recess 54a. Each of the protrusions 54 is located between the two optical fibers 30 in the circumferential direction of the irradiation probe 10. The protrusion 54 is an example of the intervening portion. The plurality of protrusions 54 is connected to each other and integrated on or near the central axis Ax1, that is, radially inward of the irradiation probe 10. Therefore, also in the example of FIG. 27, functions and effects similar to those of FIGS. 15 and 16 can be obtained.

In the example of FIG. 27, the shielding member 50 may be the reflective member that reflects leakage light from the optical fiber 30.

In the example of FIG. 27, the recess 54a and the optical fiber 30 may be in surface contact with each other, and a protrusion or a recess along the shape of the recess 54a may be formed on the outer surface 30a of the optical fiber 30 by providing a recess or a protrusion (not illustrated) in the recess 54a. In this case, the scattering region 33 in contact with the recess 54a can be formed on the outer surface 30a. The shielding member 50 is an example of the core wire, and the recess and the protrusion provided in the recess 54a are an example of a scattering body.

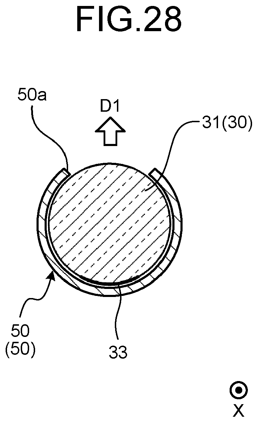

FIG. 28 is a cross-sectional view of one optical fiber 30 in the leakage portion 11. In the example of FIG. 28, the shielding member 50 partially covers the periphery of the optical fiber 30 in the leakage portion 11 and is configured as a sleeve 55 extending in the longitudinal direction. The sleeve 55 is provided with a slit-shaped opening 50a that is opened in the radial direction D1 and extends in the longitudinal direction. Also with such a configuration, the sleeve 55 is located between the two optical fibers 30 in the circumferential direction and is located radially inward of the irradiation probe 10 with respect to the optical fiber 30. Therefore, the sleeve 55 can prevent leakage light from the optical fiber 30 from traveling in the direction opposite to the radial direction D1 and the circumferential direction of the irradiation probe 10. Further, the sleeve 55 may be the reflective member that reflects leakage light from the optical fiber 30.

### (Projected Portion (Modification))

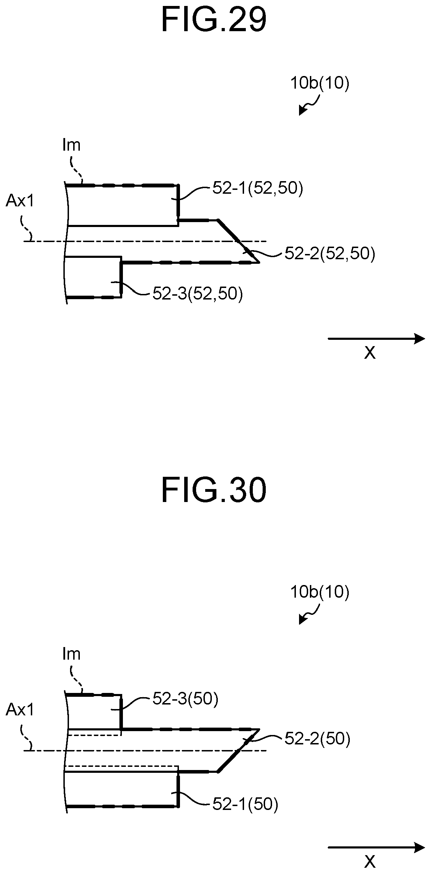

FIGS. 29 and 30 are side views of a modification of the end portion 10b of the irradiation probe 10 having a plurality of (three in this example) dummy fibers 52 as in the example of FIGS. 23 to 25. FIGS. 29 and 30 are different from each other in rotational posture around the central axis Ax1 of the irradiation probe 10. In this example, the dummy fiber 52 includes a metal member, and in a case where projection light such as X-rays is emitted from the side along the radial direction, a projection image Im as indicated by a two-dot-dash line in FIGS. 29 and 30 can be obtained. In the end portion 10b, positions and shapes in the longitudinal direction of end portions 52-1 to 52-3 of the dummy fiber 52 are set so that the rotational posture around the central axis Ax1 of the irradiation probe 10 can be determined from the projection image Im. The end portions 52-1 to 52-3 of the dummy fibers 52 are examples of a projected portion.

In a case where the projected portion has at least two portions that are separated from each other in the longitudinal direction of the irradiation probe 10 and are separated from each other in the circumferential direction of the irradiation probe 10 at a central angle different from 0° or 180° when viewed in the longitudinal direction, the projected shape of the projected portion changes according to the rotational posture. In a case where the central angle is 0° and 180°, for both of the two portions, there is a possibility of a rotational posture in which the width of the projected shape is too narrow to obtain a projected shape, and thus, the two portions are excluded.

In the examples of FIGS. 29 and 30, the end portions 52-1 to 52-3 of the three dummy fibers 52 are separated from each other in the X direction (longitudinal direction). As illustrated in FIGS. 23 to 25, the three dummy fibers 52 are separated from the central axis Ax1 in the radial direction, and are separated from each other in the circumferential direction at a central angle of 120° therebetween when viewed in the longitudinal direction. Therefore, in the examples of FIGS. 29 and 30, two end portions of the end portions 52-1 to 52-3 are examples of the two portions that exhibit functions and effects of determining the rotational posture by side projection. In this example, projection images Im different according to the rotational posture are obtained, including the rotational postures different from those in FIGS. 29 and 30. Therefore, according to the examples of FIGS. 29 and 30, it is possible to detect the rotational posture of the irradiation probe 10 relatively easily and accurately using the dummy fibers 52.

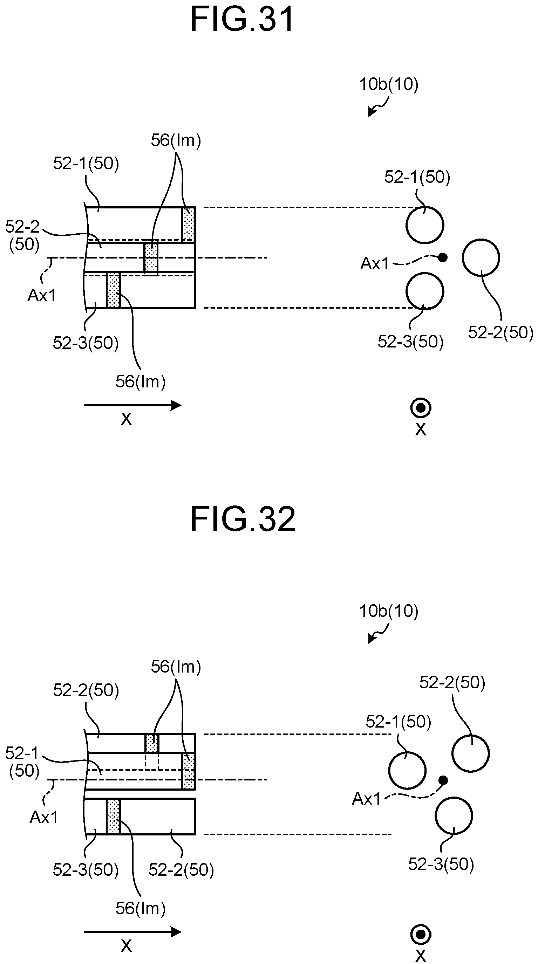

FIGS. 31 and 32 are side views (left side) and front views (right side) of a modification of the end portion 10b of the irradiation probe 10 having a plurality of (three in this example) dummy fibers 52 as in the examples of FIGS. 23 to 25. FIGS. 31 and 32 are different from each other in rotational posture around the central axis Ax1 of the irradiation probe 10. In this example, each of the dummy fibers 52 is provided with an annular cover 56 made of a metal member or the like covering the outer periphery. The dummy fiber 52 is transparent to projection light such as X-rays, and the cover 56 is opaque to the projection light. In this case, the projection image Im can be obtained for the plurality of covers 56. The arrangement of the plurality of covers 56 is set so that the rotational posture around the central axis Ax1 of the irradiation probe 10 can be determined from the projection image Im in a case where the projection light is emitted from the side along the radial direction. The cover 56 is an example of the projected portion. The cover 56 may also be referred to as a marker.

In the examples of FIGS. 31 and 32, the covers 56 provided in the end portions 52-1 to 52-3 of the three dummy fibers 52 are separated from each other in the X direction (longitudinal direction). The covers 56 provided in the three dummy fibers 52 are separated from the central axis Ax1 in the radial direction, and, as illustrated on the right side of FIGS. 31 and 32, are separated from each other in the circumferential direction at a central angle of 120° therebetween when viewed in the longitudinal direction. Therefore, in the examples of FIGS. 31 and 32, two covers of the three covers 56 are examples of the two portions that exhibit functions and effects of determining the rotational posture by side projection. In this example, projection images Im different according to the rotational posture are obtained, including the rotational postures different from those in FIGS. 31 and 32. Therefore, according to the examples of FIGS. 31 and 32, it is possible to detect the rotational posture of the irradiation probe 10 relatively easily and accurately using the dummy fibers 52 and the covers 56 thereof. In the examples of FIGS. 15 and 16, also in a case where each of the three plates 51 is given a marker (not illustrated) made of a metal member or the like so that the positions in the longitudinal direction are different from each other, the same marker arrangement as that in the examples of FIGS. 31 and 32 can be obtained, and the same functions and effects can be achieved.

In addition, the covers 56 can be arranged at predetermined intervals in the longitudinal direction. Therefore, the curved state of the irradiation probe 10 can be grasped based on the arrangement of the covers 56. In this case, the covers 56 may be arranged not only at the end portion 10b but also arranged dispersedly in a longer section of the leakage portion 11.

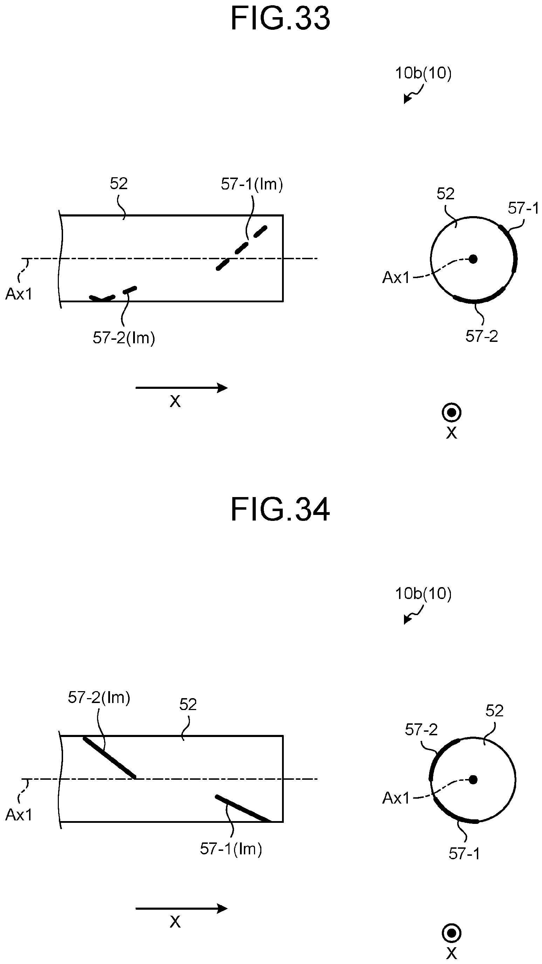

FIGS. 33 and 34 are side views (left side) and front views (right side) of a modification of the end portion 10b of the irradiation probe 10 having a dummy fiber 52 passing through the central axis Ax1 as in the examples of FIGS. 23 to 26. FIGS. 33 and 34 are different from each other in rotational posture around the central axis Ax1 of the irradiation probe 10. In this example, markers 57-1 and 57-2 made of a metal member or the like are provided on the outer periphery of the dummy fiber 52. The dummy fiber 52 is transparent to projection light such as X-rays, and the markers 57-1 and 57-2 are opaque to the projection light. In this case, the projection image Im can be obtained for the plurality of markers 57-1 and 57-2. The arrangement of the plurality of markers 57-1 and 57-2 is set so that the rotational posture around the central axis Ax1 of the irradiation probe 10 can be determined from the projection image Im in a case where the projection light is emitted from the side along the radial direction. The markers 57-1 and 57-2 are examples of the projected portion.

In the examples of FIGS. 33 and 34, the two markers 57-1 and 57-2 provided in the dummy fiber 52 are separated from each other in the X direction (longitudinal direction). The two markers 57-1 and 57-2 are separated from the central axis Ax1 in the radial direction, and, as illustrated on the right side of FIGS. 33 and 34, the central portions are separated from each other in the circumferential direction at a central angle of about 90° therebetween when viewed in the longitudinal direction. Therefore, in the examples of FIGS. 33 and 34, the two markers 57-1 and 57-2 are examples of the two portions that exhibit functions and effects of determining the rotational posture by side projection. In this example, projection images Im different according to the rotational posture are obtained, including the rotational postures different from those in FIGS. 33 and 34. Therefore, according to the examples of FIGS. 33 and 34, it is possible to detect the rotational posture of the irradiation probe 10 relatively easily and accurately using the dummy fiber 52 and the markers 57-1 and 57-2.

In addition, two portions separated from each other on the markers 57-1 and 57-2 are separated from each other in the longitudinal direction and separated from each other in the circumferential direction of the irradiation probe 10 at a central angle different from 0° or 180° when viewed in the longitudinal direction, and thus can be an example of two portions that exhibit functions and effects of determining the rotational posture by side projection.

Note that the examples of FIGS. 29 to 34 are merely examples, and the two portions and the projected portion can be implemented in various forms. For example, the two portions and the projected portion can be provided on the shielding member 50 such as the plate 51 other than the dummy fiber 52, the optical fiber 30, the cover 13, or the like, and the shapes, arrangements, and the like of the two portions and the projected portion can be variously set.

Although the embodiments of the present invention have been exemplified above, the above embodiments are merely examples, and are not intended to limit the scope of the invention. The above-described embodiments can be implemented in various other forms, and various omissions, substitutions, combinations, and changes can be made without departing from the gist of the invention. In addition, specifications (structure, type, direction, model, size, length, width, thickness, height, number, arrangement, position, material, and the like) of each configuration, shape, and the like can be appropriately changed and implemented.

For example, the irradiation probe may include both the reflective member and the non-reflective shielding member.

### Industrial Applicability

The present invention can be used in an irradiation probe and an irradiation probe system.

### Reference Signs List

- 1: IRRADIATION PROBE SYSTEM
- 10: IRRADIATION PROBE
- 10a: END PORTION
- 10b: END PORTION
- 10c: OUTER SURFACE
- 11: LEAKAGE PORTION
- 12: TRANSMISSION PORTION
- 13: COVER
- 20: DELIVERY OPTICAL FIBER
- 30, 30-1, 30-2, 30-3: OPTICAL FIBER
- 30a: OUTER SURFACE
- 31: CORE
- 31a: OUTER SURFACE
- 32: COVER LAYER
- 33: SCATTERING REGION (OUTER SCATTERING PORTION, INNER SCATTERING PORTION)
- 33a: RECESS
- 33b: PARTICLE
- 33c: HOLE
- 33d: SCATTERING ELEMENT
- 40: SWITCHING MECHANISM
- 40a: MOVABLE PORTION
- 40b-1, 40b-3: MIRROR
- 50: SHIELDING MEMBER (METAL MEMBER, PROJECTED PORTION)
- 50a: OPENING
- 51: PLATE (INTERVENING PORTION, EXTENDING PORTION)
- 52: DUMMY FIBER (OPAQUE FIBER, FIRST OPAQUE FIBER)
- 52-1 to 52-3: END PORTION (PROJECTED PORTION)
- 52a: CORE MEMBER
- 52b: COVER
- 52C: DUMMY FIBER (CORE WIRE)
- 53: DUMMY FIBER (OPAQUE FIBER, SECOND OPAQUE FIBER)
- 54: PROTRUSION
- 54a: RECESS
- 55: SLEEVE
- 56: COVER (PROJECTED PORTION)
- 57-1, 57-2: MARKER (PROJECTED PORTION)
- 100: LIGHT OUTPUT DEVICE
- 110: LIGHT SOURCE UNIT (LIGHT SOURCE)
- 200: CONTROL DEVICE (CONTROL MECHANISM, SWITCHING MECHANISM)
- 210: CONTROLLER
- 211: IRRADIATION CONTROL UNIT
- 212: INPUT CONTROL UNIT
- 213: OUTPUT CONTROL UNIT
- 214: SWITCHING CONTROL UNIT
- 220: INPUT UNIT
- 230: OUTPUT UNIT
- 241: MAIN STORAGE UNIT
- 242: AUXILIARY STORAGE DEVICE
- Ax1: CENTRAL AXIS
- Ax2: CENTRAL AXIS
- D1 to D6: RADIAL DIRECTION (OF IRRADIATION PROBE)
- Df: RADIAL DIRECTION (OF OPTICAL FIBER)
- Im: PROJECTION IMAGE
- X: DIRECTION
- Y: DIRECTION

## Claims

1. An irradiation probe comprising:
a plurality of optical fibers bundled together, each of the optical fibers including, as at least a partial section in a longitudinal direction, a leakage section configured to output leakage light radially outward, wherein
each of the optical fibers has directivity in which intensity of leakage light in a specific radial direction is higher than intensity of leakage light in another radial direction in a cross section intersecting an axial direction of the leakage section,
the optical fibers are disposed apart from a central axis of the irradiation probe in radial directions different from each other, and
the optical fibers are bundled together in a posture in which leakage light to the specific radial direction from the leakage section is directed radially outward of the irradiation probe.

2. An irradiation probe comprising:
a plurality of optical fibers bundled together, each of the optical fibers including, as at least a partial section in a longitudinal direction, a leakage section configured to output leakage light radially outward, wherein
the irradiation probe includes
a reflective member placed at least radially inward of the irradiation probe with respect to the optical fiber and configured to reflect the leakage light from the optical fiber,
each of the optical fibers has directivity in which intensity of leakage light in a specific radial direction is higher than intensity of leakage light in another radial direction in a cross section intersecting an axial direction of the leakage section,
the optical fibers are disposed apart from a central axis of the irradiation probe in radial directions different from each other, and
the optical fibers are bundled together in a posture in which leakage light to the specific radial direction from the leakage section is directed radially inward of the irradiation probe.

3. The irradiation probe according to claim 1 or 2, wherein, in the leakage section, at least one of the optical fibers includes a scattering region in which light is scattered in a predetermined range in a circumferential direction of the optical fiber.

4. The irradiation probe according to claim 3, wherein an outer surface of the optical fiber in the scattering region is a convex curved surface in which an average radius of curvature in the scattering region is equal to or greater than a radius of a general region different from the scattering region, a plane intersecting a radial direction of the optical fiber, or a concave curved surface that is concave radially inward.

5. The irradiation probe according to any one of claims 1 to 4, wherein
the optical fibers are disposed apart from a central axis of the irradiation probe in radial directions different from each other, and
the irradiation probe comprises a shielding member that prevents the leakage light from the leakage section of each of the optical fibers from traveling radially inward or a circumferential direction of the irradiation probe.

6. An irradiation probe comprising:
a plurality of optical fibers bundled together, each of the optical fibers including, as at least a partial section in a longitudinal direction, a leakage section configured to output leakage light radially outward, wherein
the optical fibers are disposed apart from a central axis of the irradiation probe in radial directions different from each other, and
the irradiation probe includes a shielding member configured to prevent the leakage light from the leakage section of each of the optical fibers from traveling radially inward or a circumferential direction of the irradiation probe.

7. The irradiation probe according to claim 5 or 6, wherein the shielding member is configured to reflect the leakage light.

8. The irradiation probe according to any one of claims 5 to 7, wherein the shielding member includes an intervening portion placed between two optical fibers of the plurality of optical fibers in a circumferential direction of the irradiation probe.

9. The irradiation probe according to any one of claims 5 to 8, wherein the shielding member includes an opaque fiber that does not transmit leakage light.

10. The irradiation probe according to claim 9, wherein the shielding member includes, as the opaque fiber, a first opaque fiber placed radially inward of the irradiation probe with respect to the optical fiber or placed between two optical fibers of the plurality of optical fibers in a circumferential direction of the irradiation probe.

11. The irradiation probe according to claim 10, wherein the shielding member includes, as the opaque fiber:
two first opaque fibers adjacent to each other; and
a second opaque fiber placed on a side close to the optical fiber with respect to a boundary between the two first opaque fibers.

12. The irradiation probe according to any one of claims 5 to 11, wherein the shielding member includes a metal member.

13. The irradiation probe according to claim 12, wherein the metal member has conductivity.

14. The irradiation probe according to claim 12 or 13, wherein the shielding member includes:
a cover as the metal member; and
a core member surrounded by the cover and made of a material having an elastic modulus smaller than an elastic modulus of the metal member.

15. The irradiation probe according to any one of claims 5 to 14, wherein the shielding member includes an extending portion extending in a radial direction of the irradiation probe at least in the leakage section.

16. The irradiation probe according to claim 15, wherein a plurality of extending portions are integrated radially inward of the irradiation probe as the extending portion.

17. The irradiation probe according to any one of claims 5 to 16, wherein the shielding member includes a sleeve configured to partially cover an outer periphery of the optical fiber at least in the leakage section and, extend in the longitudinal direction, the sleeve including an opening opened radially outward of the irradiation probe.

18. The irradiation probe according to any one of claims 5 to 17, comprising a projected portion projected by projection light of the irradiation probe, the projected portion being configured such that, when the projection light is projected in a radial direction of the irradiation probe, a projected shape by the projection light varies depending on a rotational posture around a central axis of the irradiation probe.

19. The irradiation probe according to claim 18, wherein the projected portion includes at least two portions that are separated from each other in the longitudinal direction and are separated from each other in a circumferential direction of the irradiation probe at a central angle different from 0° or 180° when viewed in the longitudinal direction.

20. The irradiation probe according to any one of claims 1 to 19, wherein the optical fiber includes an outer scattering portion placed radially outward of the irradiation probe in the leakage section to scatter light.

21. The irradiation probe according to any one of claims 1 to 20, wherein the optical fiber includes an inner scattering portion placed radially inward of the irradiation probe in the leakage section to scatter light.

22. The irradiation probe according to any one of claims 1 to 21, comprising
a core wire extending in a longitudinal direction of the irradiation probe, wherein
the plurality of optical fibers are arranged along an outer periphery of the core wire.

23. The irradiation probe according to claim 22, wherein a diameter of the core wire is larger than a diameter of the optical fiber.

24. The irradiation probe according to claim 22 or 23, wherein the plurality of optical fibers are at least partially accommodated in a recess provided in the core wire.

25. The irradiation probe according to any one of claims 22 to 24, wherein the core wire includes a portion made of a synthetic resin material.

26. The irradiation probe according to any one of claims 22 to 25, wherein the core wire includes a scattering body.

27. An irradiation probe system comprising:
the irradiation probe according to any one of claims 1 to 26;
a light source; and
a switching mechanism configured to selectively input light from the light source to at least one of the plurality of optical fibers.

28. An irradiation probe system comprising:
an irradiation probe including
a plurality of optical fibers bundled together, the plurality of optical fibers being arranged side by side in a circumferential direction of the irradiation probe and configured to leak light radially outward from an outer surface of each of the plurality of optical fibers in a leakage section in a longitudinal direction;
a light source; and
a switching mechanism configured to selectively input light from the light source to at least one of the plurality of optical fibers.

29. The irradiation probe system according to claim 27 or 28, comprising a plurality of light sources as the light source.

30. The irradiation probe system according to any one of claims 27 to 29, comprising a control mechanism configured to control at least one of the light source and the switching mechanism so that light from the light source is intermittently input to the optical fiber.

31. The irradiation probe system according to any one of claims 27 to 30, wherein the switching mechanism is configured to operate so that the optical fiber to which light from the light source is input is sequentially switched over time in a circumferential direction of the irradiation probe.
